# EUROPÄISCHE PATENTANMELDUNG

(11) **EP 2 468 405 A2**
(43) Veröffentlichungstag der Anmeldung: **27.06.2012**
(21) Anmeldenummer: 11010000.5
(22) Anmeldetag: 20.12.2011
(51) Int. Cl.: B01L 5/02

(54) **Adapter zur Verbindung eines Sterilfilters mit einem Nährmedienbehälter zum Zwecke der Funktionsprüfung des Sterilfilters**

(30) Priorität: 24.12.2010 DE 102010056292
(71) Anmelder: K+S Aktiengesellschaft, 34131 Kassel (DE)
(72) Erfinder: Natelberg, Torsten, 51065 Köln (DE); Majewski, Elke, 34123 Kassel (DE); Bräutigam, Burkhard, 34560 Fritzlar (DE)

(57) **Zusammenfassung**

Gegenstand der Erfindung ist ein Adapter (1) zur Verbindung eines Sterilfilters mit einem Nährmedienbehälter (20) zum Zwecke der Funktionsprüfung des Sterilfilters, wobei der Adapter (1) einen Anschluss zur Aufnahme durch den Anschlussstutzen (22) des Nährmedienbehälters (20) und einen Anschluss für den Sterilfilter (10) aufweist, wobei der Adapter (1) ein Tauchrohr (6) aufweist, das bis in das Nährmedium (23) hineinragt, und wobei neben dem Tauchrohr (6) der Auslass (9) mit seinem einen Ende mit dem Gasraum des Nährmedienbehälters (20) in Verbindung steht.

## Beschreibung

Die Erfindung betrifft einen Adapter zur Verbindung eines Sterilfilters mit einem Nährmedienbehälter zum Zwecke der Funktionsprüfung eines Sterilfilters.

Die vorliegende Erfindung dient dem Zweck der Funktionsprüfung von Sterilfiltern, die für die Herstellung steriler Luft bzw. einem sterilen Gas für die Kultivierung von Mikroorganismen eingesetzt werden.

Sicherzustellen ist über die Zeit die Funktionstüchtigkeit eines solchen Sterilfilters. Für eine Überprüfung der Funktion sind bisher Testsysteme im Einsatz, die den transmembranen Druck messen und durch Detektion von Druckänderungen Leckagen im Filter erkennen. Durch Leckagen, die z. B. durch Erweiterungen der Porengröße in der Membran bedingt sind, können Mikroorganismen in den steril zu haltenden Raum jenseits des Filters gelangen und dort zu einer unerwünschten Kontamination führen. Für die Funktionsprüfung gilt die sogenannte FDA-Richtlinie (Guideline on sterile drug products produced by aseptic processing 1987).

Darüber hinaus sind sogenannte Integritätsprüfungen von Filtern bekannt, die einerseits destruktiv und andererseits nicht destruktiv durchgeführt werden können. Destruktive Tests werden meist stichprobenhaft und statistisch signifikant an Chargen vorgenommen, wohingegen nicht destruktive Tests mit jedem Filter vor, bzw. nach dessen Anwendung oder in regelmäßigen Abständen durchgeführt werden. Hierbei gibt es verschiedene Arten nicht destruktiver Tests, den sogenannten Bubble-Point Test, den Diffusionstest sowie für hydrophobe Membranfilter den Wasserfluss-Integritätstest. Der Druckhalte-, Forward-Flow-, und der Druckabfalltest stellen Varianten des bereits zuvor erwähnten Diffusionstests dar. Hierbei wird vorausgesetzt, dass ein spezifischer physikalischer Parameter mit dem Bakterienrückhaltevermögen korreliert und damit als definiertes Kriterium der Filterintegrität dienen kann.

Die bekannten Testverfahrensprüfungen setzen einen erheblichen apparativen Aufwand voraus. Die Investitionskosten für bekannte Filterintegritätstestgeräte liegen zwischen EUR 4.000,-- und ca. EUR 50.000,--. Die Standzeit solcher Sterilfilter ist abhängig von der Belastung. Da diese durchaus unterschiedlich sein kann, besteht ein besonderes Interesse daran, zu ermitteln, wann die Standzeit abgelaufen ist.

Darüber hinaus ist ein Verfahren zur Überprüfung eines Sterilfilters bekannt, bei dem der Sterilfilter gasführend mit dem Nährmedium in einem Nährmedienbehälter in Verbindung steht. Zum Zwecke der Funktionsprüfung des Sterilfilters wird über einen vorbestimmten Zeitraum ein Gas durch den Sterilfilter und das Nährmedium hindurchgesaugt, wobei nach einer vorbestimmten Zeit die Sterilität des Nährmediums überprüft wird. In Anbetracht der Kontaminanten in dem Nährmedium lassen sich Rückschlüsse auf die Funktionsfähigkeit des Sterilfilters ziehen; denn weist der Sterilfilter ein Leck auf, so werden Kontaminanten durch das angesaugte Gas z. B. Umgebungsluft in das Nährmedium eingetragen. Diese können dann gegebenenfalls nach entsprechender Vermehrung nachgewiesen werden. Insofern steht ein einfaches und kostengünstiges Verfahren zur Überprüfung der Funktionsfähigkeit eines Sterilfilters zur Verfügung.

Gegenstand der Erfindung ist nun ein Adapter zur Verbindung eines Sterilfilters mit einem Nährmedienbehälter zum Zwecke der Funktionsprüfung des Sterilfilters. Der Adapter zeichnet sich im Einzelnen dadurch aus, dass der Adapter einen Anschluss zur Aufnahme durch den Anschlussstutzen des Nährmedienbehälters und einen Anschluss für den Sterilfilter aufweist, wobei der Adapter ein Tauchrohr aufweist, das bis in das Nährmedium hineinragt, und wobei neben dem Tauchrohr der Auslass mit seinem einen Ende mit dem Innenraum (Gasraum) des Nährmedienbehälters in Verbindung steht. Hieraus wird Folgendes deutlich. Der Adapter stellt die Verbindung zwischen dem Sterilfilter einerseits und dem Nährmedienbehälter andererseits dar. Der Adapter weist insofern ein Tauchrohr auf, das bis in das Nährmedium in dem Nährmedienbehälter hineinragt, wobei ein Auslass vorgesehen ist, der in den Gasraum des Nährmedienbehälters mündet. Wird somit der Auslass mit einem Unterdruck beaufschlagt, wie dies beispielsweise der Fall ist, wenn eine Pumpe an den Auslass angeschlossen wird, dann wird aufgrund des sich bildenden Unterdrucks im Gasraum des Nährmedienbehälters über das Tauchrohr durch das Nährmedium hindurch beispielsweise Umgebungsluft durch den Sterilfilter angesaugt. Ist der Sterilfilter nicht mehr funktionstüchtig, dann werden Kontaminanten in das Nährmedium eingetragen, wobei die Kontamination insofern gegebenenfalls nach Vermehrung der Kontaminanten nachweisbar ist, und insofern Rückschlüsse auf die Funktionstüchtigkeit des Sterilfilters gezogen werden können.

Nach einem weiteren Merkmal der Erfindung ist vorgesehen, dass der Durchmesser des Tauchrohres kleiner ist als der Innendurchmesser des Anschlussstutzens des Nährmedienbehälters, wobei der Auslass mit seinem einen Ende in den Raum zwischen Tauchrohr und Innenwandung des Anschlussstutzens mündet. Das heißt, zwischen Tauchrohr einerseits und dem Innendurchmesser des Anschlussstutzens des Nährmedienbehälters besteht ein Freiraum, in den der Auslass des Adapters mündet.

Nach einem weiteren Merkmal der Erfindung ist vorgesehen, dass der Auslass dicht durch eine Sterilbarriere, insbesondere durch ein Ventil oder einen Filter, gegen den Eintritt von Bakterien verschließbar ist. Hierdurch wird sichergestellt, dass dann, wenn nach der Sterilisation die Vakuumpumpe noch nicht an dem Auslass angeschlossen worden ist, nicht durch den Auslass in den Nährmedienbehälter Kontaminanten eindringen können.

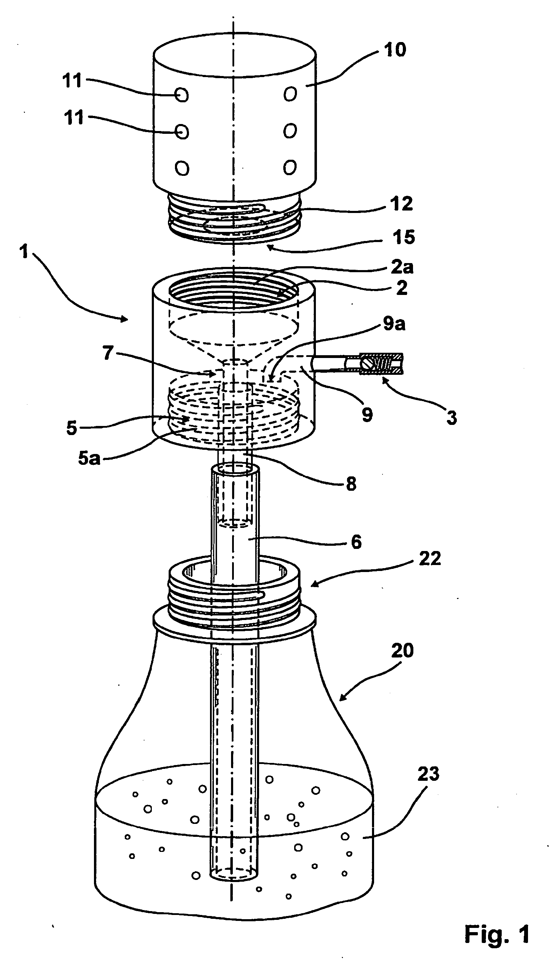

Anhand der Zeichnung wird die Erfindung nachstehend beispielhaft näher erläutert.

So zeigt die einzige Figur, eine schematische Darstellung der gesamten Anordnung umfassend den Sterilfilter, den Adapter und den Nährmedienbehälter in Form einer Flasche. Der Sterilfilter ist im dargestellten Fall als Filterkerze ausgebildet, die in einem Gehäuse mit Gasdurchlassöffnungen 11 angeordnet ist. Eine solche Filterkerze besitzt zur Reinigung des Gases Kapillaren oder Membranlamellen, die von dem Gas durchströmt werden, wenn dieses durch Gaseintrittsöffnungen 11 strömt. Zentrisch besitzt der Sterilfilter einen Kanal 15 in den das gereinigte Gas geleitet wird, der im Bereich des Gewindes 12 ausläuft. Der mit 1 bezeichnete Adapter weist auf seiner Oberseite einen Anschluss 2 mit ein im Innengewinde 2a zur Aufnahme des Außengewindes 12 des mit 10 bezeichneten Sterilfilters auf. Denkbar ist auch eine Verbindung mittels Bajonettverschluss oder mittels Steckverbindung. Auf der Unterseite ist der Adapter 1 ebenfalls mit einem Anschluss 5 mit einem Innengewinde 5a versehen, was der Aufnahme des mit einem Außengewinde versehenen Anschlussstutzens 22 des Nährmedienbehälters 20 dient. In dem Nährmedienbehälter 20 befindet sich das Nährmedium 23.

Der Adapter 1 besitzt einen mittigen Durchlass 7, wobei in den Durchlass ein Rohr 8 eingesetzt ist, auf das das mit 6 bezeichnete Tauchrohr aufsetzbar ist. Alternativ kann das Tauchrohr auch direkt eingesetzt werden.

Im Bereich des Innengewindes 5 des Adapters 1 befindet sich beabstandet einerseits zum Außenumfang des Rohres 8 und zum Innendurchmesser des Innengewindes 5 die Mündung 9a des mit 9 bezeichneten Auslasses. Die Mündung 9a befindet sich somit im Gasraum des Nährmedienbehälters. Der mit 9 bezeichnete Auslass umfasst als Sterilbarriere ein federbelastetes Rückschlagventil 3, das dafür sorgt, dass nach Entfernung der Vakuumpumpe vom Auslass 9 keine Umgebungsluft in den Gasraum des Nährmedienbehälters eintreten kann. Alternativ kann auch ein steriler Einweg-Filter eingesetzt werden, der verhindert, dass Kontaminanten in die Nährflüssigkeit gelangen.

Der mit 10 bezeichnete Sterilfilter weist auf seinem äußeren Umfang eine ganze Reihe von umfangsverteilt angeordneten Einlässen 11 auf, die durch eine Filterkerze (nicht dargestellt) mit einem zentrischen Durchlass 15 in Verbindung stehen. Die Umgebungsluft wird durch die am Auslass 9a angeschlossene Vakuumpumpe (nicht dargestellt) durch das Nährmedium hindurch angesaugt wird. Die angesaugte Umgebungsluft wird zunächst im Sterilfilter 10 gereinigt, bevor sie durch den Adapter 1 hindurch in das Nährmedium 23 gelangt. Die angesaugte gereinigte Umgebungsluft steigt durch das Nährmedium 23 auf, und wird durch den Auslass 9 nach außen abgesaugt. Hierbei arbeitet die an dem Auslass 9 angeschlossene Vakuumpumpe gegen das federbelastete Ventil 3. Sobald die Vakuumpumpe abgeschaltet ist, verschließt sich die Öffnung im Auslass durch das Ventil 3 oder durch einen Einwegfilter, die als Sterilbarriere fungieren. Nach einer vorbestimmbaren Zeit wird dann das Nährmedium 23 des Nährmedienbehälters 20 in Hinblick auf eine Kontamination untersucht, um hierdurch zu kontrollieren, ob der Sterilfilter 10 noch funktionsfähig ist. Die Funktion des Ventils 3 oder des Filters wird auch dann benötigt, wenn nach der Sterilisation der gesamten Vorrichtung, vor dem Anschließen der Vakuumpumpe, die Vorrichtung mit nicht sterilen Räumen in Kontakt kommt.

### Bezugszeichenliste:

- 1: Adapter
- 2: Anschluss
- 2a: Innengewinde
- 3: Ventil
- 5: Anschluss
- 5a: Innengewinde
- 6: Tauchrohr
- 7: Durchlass
- 8: Rohr
- 9: Auslass
- 9a: Mündung
- 10: Sterilfilter
- 11: Einlässe
- 12: Außengewinde
- 15: zentrischer Durchlass im Sterilfilter
- 20: Nährmedienbehälter
- 22: Anschlussstutzen
- 23: Nährmedium

## Patentansprüche

1. Adapter (1) zur Verbindung eines Sterilfilters mit einem Nährmedienbehälter (20) zum Zwecke der Funktionsprüfung des Sterilfilters,
**dadurch gekennzeichnet,**
**dass** der Adapter (1) einen Anschluss (2) zur Aufnahme durch den Anschlussstutzen (22) des Nährmedienbehälters (20) und einen Anschluss (5) für den Sterilfilter (10) aufweist, wobei der Adapter (1) ein Tauchrohr (6) aufweist, das bis in das Nährmedium (23) hineinragt, und wobei neben dem Tauchrohr (6) der Auslass (9) mit seinem einen Ende mit dem Gasraum des Nährmedienbehälters (20) in Verbindung steht.

2. Adapter nach Anspruch 1,
**dadurch gekennzeichnet,**
**dass** der Durchmesser des Tauchrohres (6) kleiner ist, als der Innendurchmesser des Anschlussstutzens (22) des Nährmedienbehälters (20), wobei der Auslass (9) mit seinem einen Ende in den Raum zwischen Tauchrohr (6) und Innenwandung des Anschlussstutzens (22) mündet.

3. Adapter nach einem der voranstehenden Ansprüche,
**dadurch gekennzeichnet,**
**dass** der Auslass (9) eine Sterilbarriere aufweist.

4. Adapter nach Anspruch 3,
**dadurch gekennzeichnet,**
**dass** der Auslass (9) ein Ventil (3) aufweist.

5. Adapter nach Anspruch 3,
**dadurch gekennzeichnet,**
**dass** der Auslass (9) einen Filter, insbesondere einen sterilen Einwegfilter aufweist.

6. Adapter nach einem der voranstehenden Ansprüche,
**dadurch gekennzeichnet,**
**dass** der Sterilfilter (10) auf dem Außenumfang Gaseintrittsöffnungen (11) aufweist, die zentrisch in einen Kanal (15) münden.
